Europäisches Patentamt

European Patent Office

Office européen des brevets

(11) Veröffentlichungsnummer: **0 071 131**
A2

(12) **EUROPÄISCHE PATENTANMELDUNG**

(21) Anmeldenummer: 82106462.3

(22) Anmeldetag: 17.07.82

(51) Int. Cl.³: **A 61 N 1/36**
G 08 C 19/00

(30) Priorität: 30.07.81 DE 3130104

(43) Veröffentlichungstag der Anmeldung:
09.02.83 Patentblatt 83/6

(84) Benannte Vertragsstaaten:
AT BE CH DE FR GB IT LI NL SE

(71) Anmelder: Deutsche Nemectron GmbH
Durlacher Allee 47
D-7500 Karlsruhe 1(DE)

(72) Erfinder: Hildebrandt, Jürgen, Dipl. Phys.
Niobestrasse 22
D-8000 München 82(DE)

(74) Vertreter: Patentanwälte Dr.-Ing. Hans Lichti Dipl.-Ing.
Heiner Lichti Dipl.-Phys. Dr.Jost Lempert
Postfach 410760 Durlacherstrasse 31
D-7500 Karlsruhe 41(Grötzingen)(DE)

(54) Vorrichtung zur Stimulation eines menschlichen Muskels.

(57) Bei einer Vorrichtung zur Stimulation eines menschlichen Muskels im Hinblick auf die Überwindung von Lähmungserscheinungen od. dgl. bekannter Art können willkürlich gesteuerte Muskelspiele nur mit erheblichen Einschränkungen und ausschließlich aufgrund manueller Eingriffe erfolgen. Im Hinblick auf eine größere Vielzahl von willkürlichen, von manuellen Eingriffen freien Funktionen wird eine Vorrichtung mit den folgenden Merkmalen vorgeschlagen:

a) Mehrere separate in einem Muskel implantierbare Reizgeber mit Elektroden, von denen jeder mit dem Empfänger, der ein für ihn bestimmtes Signal selektiv empfängt, mit einem Reizgenerator, dessen Reizsignal in seiner Amplitude, Frequenz und Dauer mittels eines Empfangssignals gesteuert ist, und mit einer Speiseeinrichtung versehen ist, die mit empfangener hochfrequenter Energie versorgt ist.

b) Implantierbare Muskelpotentialsensoren, von denen jeder mit einem Sender versehen ist, der ein von einem gemessenen Muskelpotential moduliertes und selektiv auswertbares Signal ausstrahlt und von denen jeder eine Speiseeinrichtung aufweist, die ebenfalls mit empfangener hochfrequenter Energie versorgt ist.

c) Einen den Sendern der Muskelpotentialsensoren zugeordneten Telemetrieempfänger, der über eine datenverarbeitende Schaltung einen den Reizgebern zugeordneten Telemetriesender moduliert.

FIG. 1

DR. ING. HANS LICHTI · DIPL.-ING. HEINER LIC 0071131
DIPL.-PHYS. DR. JOST LEMPERT
PATENTANWÄLTE

D-7500 KARLSRUHE 41 (GRÖTZINGEN) · DURLACHER STR. 31 (HOCHHAUS)
TELEFON (0721) 48511

Deutsche Nemectron GmbH
Durlacher Allee 47

D-7500 Karlsruhe 1

6455/82

13. Juli 1982

Vorrichtung zur Stimulation eines menschlichen Muskels

Die Erfindung betrifft eine Vorrichtung zur Stimulation eines menschlichen Muskels, mit mindestens einem im Muskel implantierbaren Reizgeber, mit einem Empfänger und einer Speiseeinrichtung, mit mehreren Elektroden zur Abgabe der Reizsignale, die dem Muskel an verschiedenen Stellen hinsichtlich der Impulsfolge, der Impulsamplitude, der Impulsdauer und der Impulsform genau definierte Reizimpulse zuführen, mit denen eine möglichst natürlich verlaufende Muskelkontraktion hervorgerufen wird.

Eine derartige Vorrichtung dient insbesondere zur Überwindung von Lähmungserscheinungen od.dgl. physiologischer Art. Es ist eine programmierbare Stimulationsvorrichtung für menschliches Gewebe bekannt, bei dem mit Hilfe eines externen Steuergerätes und eines implantierten Empfängers an mehreren über Leitungen mit einem Reizgeber verbundenen, an zu reizenden Stellen im Gewebe implantierten Elektroden von dem Reizgeber vorprogrammierbare Reizsignale in zyklischer Folge erzeugt werden. Mit derartigen Einrichtungen kann bei geeigneter Programmierung ein beabsichtigtes Muskelspiel für einen bestimmten vorgesehenen Bewegungsablauf durch Betätigung eines Schalters hervorgerufen und wiederholt werden. Durch Programmänderung sind ferner auch abweichende Bewegungsabläufe einstellbar. Sowohl für die Auslösung als auch für den Ablauf der Muskelreaktion ist eine Betätigung von Schalt- oder Einstellorganen durch den Patienten oder behandelnden Arzt erforderlich. Ferner sind vornehmlich die erforderlichen implantierten Leitungen, die die Reizelektroden mit dem Reizgeber verbinden, im Dauerbetrieb störanfällig. Bei der bekannten Vorrichtung kann der Patient auch nur eine kleine Steuereinheit mitnehmen und betätigen, die lediglich sehr beschränkte Bewegungsreizungen zuläßt, während vielfältigere Bewegungsabläufe nur über ein stationäres, nicht transportables Steuergerät erzeugt werden können. Die Schaltung dieser bekannten Vorrichtung ist im einzelnen in der DE-OS 28 03 366 beschrieben. Die bekannte Vorrichtung eignet sich insgesamt nur mit erheblichen Einschränkungen zur Erzeugung willkürlich gesteuerter Muskelspiele.

Demgegenüber besteht die Aufgabe der Erfindung darin, eine eingangs genannte Anordnung so auszubilden, daß eine willkürliche, von manuellen Eingriffen freie Funktion ermöglicht ist, bei der im Muskel geführte implantierte Leitungen entfallen und die dennoch eine völlig freie Disposition von Reizelektroden ermöglicht.

Diese Aufgabe ist für eine Vorrichtung zur Stimulation eines menschlichen Muskels erfindungsgemäß gelöst durch eine Vorrichtung mit folgender Merkmalskombination:

a) Mehrere separate in einem Muskel implantierbare Reizgeber mit Elektroden, von denen jeder mit dem Empfänger, der ein für ihn bestimmtes Signal selektiv empfängt, mit einem Reizgenerator, dessen Reizsignal in seiner Amplitude, Frequenz und Dauer mittels eines Empfangssignals gesteuert ist, und mit einer Speiseeinrichtung versehen ist, die mit empfangener hochfrequenter Energie versorgt ist.

b) Implantierbare Muskelpotentialsensoren, von denen jeder mit einem Sender versehen ist, der ein von einem gemessenen Muskelpotential moduliertes und selektiv auswertbares Signal ausstrahlt und von denen jeder eine Speiseeinrichtung aufweist, die ebenfalls mit empfangener hochfrequenter Energie versorgt ist.

c) Einen den Sender der Muskelpotentialsensoren zugeordneten Telemetrieempfänger, der über eine datenverarbeitende Schaltung einen den Reizgebern zugeordneten Telemetriesender moduliert.

Man geht also derart vor, daß mittels mehrerer an verschiedenen Orten im Muskel implantierter Muskelpotentialsensoren das jeweils gegebene Muskelpotential gemessen und über vorzugsweise mit den Potentialsensoren integrierte Sender an einen Telemetrieempfänger gesendet wird, daß die empfangene Signale in einer datenverarbeitenden Schaltung verarbeitet und die verarbeiteten Signale über einen Telemetriesender mehreren ebenfalls verteilt im Muskel implantierten zugeordneten Reizgebern übersandt wird, wodurch diese Reizimpulse über Elektroden abgeben.

Erfindungsgemäß werden also Sensoren vorgesehen, die die Muskelpotentiale messen. Aufgrund dieser gemessenen Muskelpotentiale werden dann die Reizgeber selektiv angesteuert und die Bewegung des Muskels bewirkt über Reizelektroden, die in Abhängigkeit von gemessenen Muskelpotentialen mit physiologischen oder programmierbaren Reizsignalen selektiv und definiert versorgt sind. Durch das
Vorsehen von Sensoren wird eine manuelle Steuerung, auch wenn
diese beispielsweise über einen externen Rechner erfolgt, vermieden
und eine Bewegungsablaufssteuerung in Abhängigkeit von den
elektrophysiologischen Gegebenenheiten des Muskels erreicht,
wobei insbesondere durch die Mehrzahl der von einander getrennten
Reizgeber eine optimale Anpassung und Manigfaltigkeit der Bewegungsabläufe erzielbar ist. Zusätzliche Verbindungsleitungen zwischen Reizgebern und Elektroden können vermieden werden; die Elektroden können
direkt mit den Reizgebern verbunden sein.

Gemäß einer bevorzugten Ausgestaltung ist vorgesehen, daß die
Speiseeinrichtungen mit Sendeenergie des Telemetriesenders betrieben
sind. Grundsätzlich könnten die Speiseeinrichtungen der Reizgeber
und der Muskelpotentialsensoren auch von separaten Energiesendern
mit Energie versehen werden, was aber einen erhöhten Aufwand bedeuten
würde, der durch die genannte Ausführungsform in vorteilhafter Weise
vermieden wird.

Gemäß einer anderen bevorzugten Ausgestaltung ist vorgesehen, daß
den Sendern der Muskelpotentialsensoren und dem Telemetrieempfänger
ein gegenüber den Empfängern der Reizgeneratoren und dem Telemetriesender unterschiedliches Frequenzband zugeordnet ist. Hierdurch wird
eine zuverlässige Trennung der unverarbeiteten, von den Muskelpotentialsensoren abgegebenen Signale und der nach deren Verarbeitung vom
Telemetriesender für die Empfänger der Reizgeneratoren abgesandten
Signale erreicht und eine gegenseitige Störung oder Beeinflussung
zuverlässig vermieden.

Gemäß einer weiteren bevorzugten Ausgestaltung ist vorgesehen, daß
die Antennen des Telemetrieempfängers und des Telemetriesenders in einer aufsetzbaren Einheit angeordnet sind, die die an einem den zu stimulierenden Muskel aufweisenden Körperglied unter mindestens partieller Bedeckung der Gliedmaße anbringbar ist, wobei diese Ausgestaltung dadurch weitergebildet sein kann, daß die Antennen von Telemetrieempfänger und -sender in einer Manschette angeordnet sind, die das Körperglied umgeben kann. Hierdurch wird dem Patienten ein bequemes Tragen der externen, nicht implantierten Teile der Vorrichtung ermöglicht.

Weitere Vorteile und Merkmale der Erfindung ergeben sich aus den Ansprüchen und aus der nachfolgenden Beschreibung, in der unter Bezugnahme auf die schematisch vereinfacht dargestellte Zeichnung ein Ausführungsbeispiel der Erfindung im einzelnen erläutert wird. Dabei zeigt:

Figur 1    ein Blockschaltbild der gesamten Vorrichtung mit schematischer Darstellung eines Muskels;

Figur 2    einen Querschnitt durch einen Reizgeber der Vorrichtung;

Figur 3    einen Querschnitt durch einen Muskelpotentialsensor der Vorrichtung; und

Figur 4    ein Beispiel der Folge einer Informationscodierung für die Reizgeber, mit Spannungsverlauf der Elektrode eines Reizgebers.

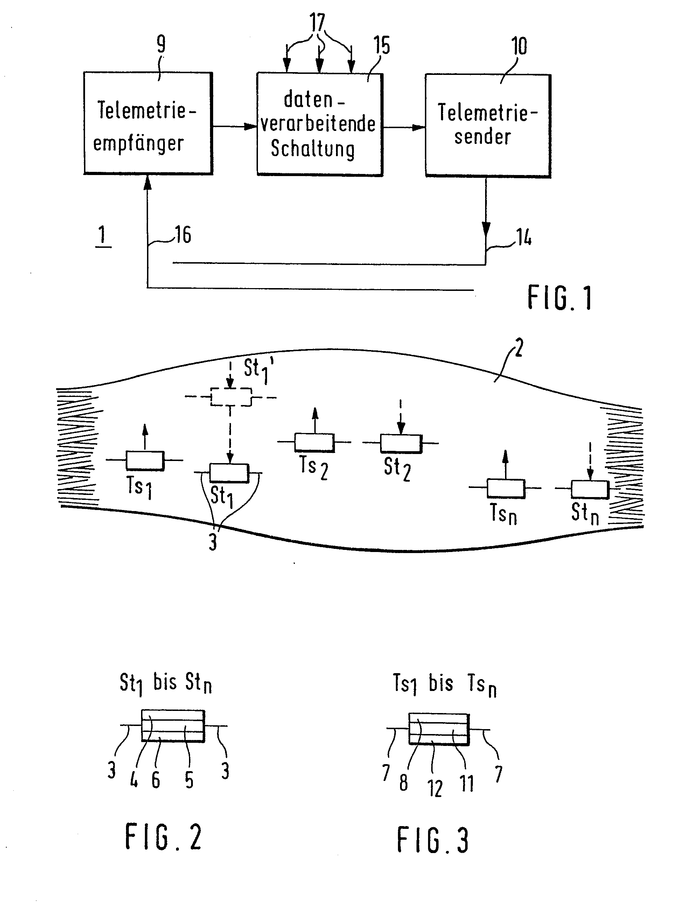

In der Figur 1 sind im Blockschaltbild $\underline{1}$ zur Stimulation eines menschlichen Muskels $\underline{2}$ Reizgeber $St_1$ bis $St_n$ an frei wählbaren Stellen im Muskel implantiert angeordnet, denen jeweils ein Muskelpotential-sensor $Ts_1$ bis $Ts_n$ zugeordnet ist, die ebenfalls im Muskel implantiert sind; d.h. also, je ein Muskelpotentialsensor Ts und mindestens ein Reizgeber St bilden eine Funktionseinheit, die mit Hilfe später beschriebener Einrichtungen selektiv betreibbar ist. Auch die Zuordnung mehrerer gleichartiger Reizgeber $St_1'$ bis $St_n'$ zu einem Muskelpotentialsensor Ts oder mehrerer Muskelpotentialsensoren $Ts_1$ bis $Ts_n$ zu einem Reizgeber St ist im Bedarfsfalle möglich, wobei einander zugeordnete Sensoren Ts und Reizgeber St räumlich miteinander verbunden, insbesondere in einem gemeinsamen Gehäuse angeordnet sein können. Sowohl die Reizgeber St als auch die Muskelpotentialsensoren Ts sind nach freier Wahl des behandelnden Arztes placierbar. Sie sind leitungslos, d.h. Reizelektroden 3 und Sensorelektroden 7 sind unmittelbar am Reizgeber St bzw. Sensor Ts selbst angeordnet (vgl. auch Fig. 2 und 3). Die Reizgeber St werden von einem weiter unten beschriebenen Telemetriesender 10 mit modulierter hochfrequenter Energie versorgt, der auch mittels einer im Reizgeber St integrierten Speiseeinrichtung 6 den Energiebedarf des Empfängers 4 und des Reizgenerators 5 deckt. Entgegen der Darstellung kann bei einer praktischen Ausführungsform eines Reizgebers St der Empfänger 4, der Reizgenerator 5 und die Speiseeinrichtung 6 in bekannter Weise aus einer einzigen integrierten Schaltung bestehen, deren Empfangsschwingkreis eine auf die Frequenz des Telemetriesenders 10 abgestimmte Antenne enthält.

Die ferner im Muskel 2 implantierten Muskelpotentialsensoren $Ts_1$ bis $Ts_n$ sind mit Sensorelektroden 7 versehen, mit denen an geeigneten Stellen eines nervengeschädigten Muskels noch vorhandene Muskelpotentiale abgreifbar sind, die mittels eines Verstärkers und Modulators 11 zu Steuersignalen für einen in einem Muskelpotentialsensor Ts integrierten Telemetriesender 8 aufbereitet werden. Ähnlich den Reiz-gebern St sind die Muskelpotentialsensoren Ts mit einer Speiseeinrichtung 12 versehen, die mittels eines zugeordneten Empfängers oder mittels ihres als Sende-Empfänger ausgebildeten Telemetriesenders 8 von dem externen Telemetriesender 10 mit Energie versorgt sind. Ergänzend gelten analog die Ausführungen zum Aufbau der Reizgeber St.

Zum Betrieb der implantierten Reizgeber St und der implantierten Muskelpotentialsensoren Ts dient ein Telemetriesender 10 grundsätzlich bekannter Bauart, dessen Sendeantenne 14 beispielsweise in einer den Muskel 2 bzw. die zugehörigen Gliedmaße umgebenden und in der Zeichnung zur Vereinfachung nicht gezeigten Manschette oder in einer aufsetzbaren Vorrichtung - beispielsweise in Form einer Hohlkugelkalotte   in der Gesäßpartie der Hose - angeordnet ist, die auch eine Sendeantenne 16 für einen Telemetrieempfänger 9 bzw. eine einzige in bekannter Weise umschaltbare Antenne enthält. Telemetrieempfäner 9, -Sender 10 und eine datenverarbeitende Schaltung 15 können ebenso wie eine Batterie beispielsweise an einem Gürtel getragen werden und sind  über Koaxialkabel mit den Antennen 14, 16 verbunden.

Der Telemetriesender 10 sendet Signale aus, die von den Reizgebern $St_1$ bis $St_n$ selektiv empfangen werden, wobei in den Signalpausen die Sendeenergie des Telemetriesenders 10 zur Energieversorgung der Speiseeinrichtungen 6 und 12 der Reizgeber $St_1$ bis $St_n$ und der Muskelpotentialsensoren $Ts_1$ bis $Ts_n$ benutzt wird, wobei hierzu die Sendehochfrequenz gleichgerichtet und gespeichert wird.

Die Steuerung des Telemetriesenders 10 und dessen Modulation erfolgt mit Hilfe einer datenverarbeitenden Schaltung 15, deren Kern ein Mikroprocessor sein kann und in der die mittels eines Telemetrieempfängers 9 empfangenen Signale von den Sendern 8 der Muskel-

potentialsensoren Ts zur Ansteuerung des Telemetriesenders 10 aufbereitet werden.

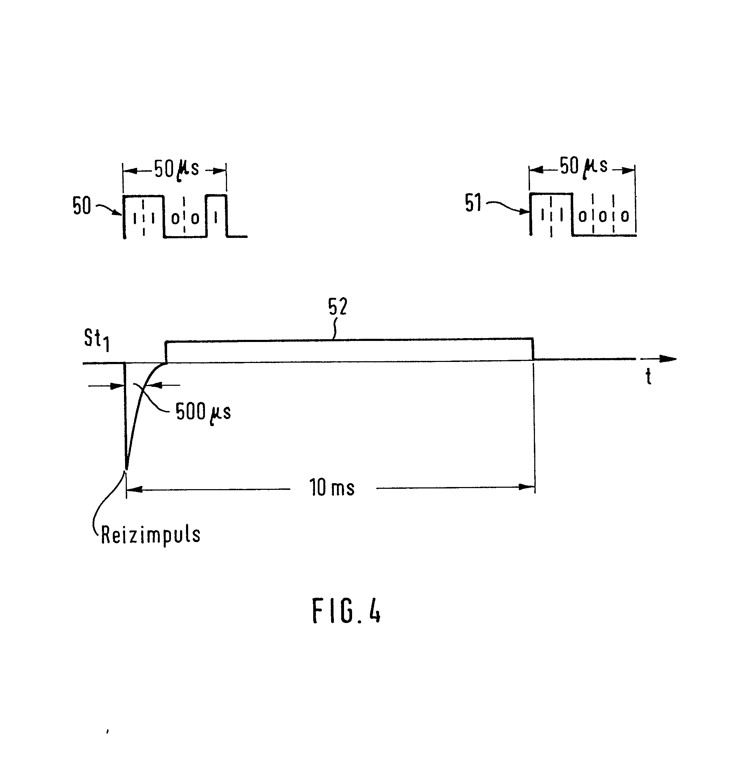

Die den Sender 10 steuernde datenverarbeitende Schaltung 15 bildet aus den Signalen des Telemetrieempfängers 9, der alle Signale der von den Sendern 8 der Muskelpotentialsensoren Ts gesendeten Signalfolge demoduliert und decodiert, in einer Codierstufe verstärkte und addressierte Einzelsignale. Zur ggfls. therapeutisch gewünschten Erweiterung dieser Einzelsignale sind an der datenverarbeitenden Schaltung 15 Eingänge 17 für Regelspannungen vorgesehen, mit denen der Arzt oder der Patient zusätzlich einen Einfluß auf die Impulsamplitude, die Impulsdauer und/oder die Impulsform vornehmen kann. Mit Hilfe der addressierten und geformten Einzelsignale erfolgt eine Steuerung des Telemetriesenders 10 derart, daß den Einzelsignalen proportionale Signale selektiv den Reizgebern zugeführt werden. Im Empfänger 4 des Reizgebers St wird beispielsweise ein vom Telemetriesender 10 ausgesandtes moduliertes HF-Signal empfangen. Die Information des Signals wird zur Erzeugung von Reizimpulsen im Reizgenerator 5 und Abgabe derselben über die Elektroden 3 genutzt. Die Information kann jeweils aus einem 5 Bit langen Code-Wort 50, 51 von beispielsweise je 50 Mikrosekunden bestehen, wie es in der Figur 4 dargestellt ist. Eine solche Codierung eignet sich für eine 8-Kanal-Vorrichtung, die also bis zu 8 Reizgeber St aufweist. Das erste Bit dient zur Synchronisation. Die nächsten 3 Bits dienen der Auswahl des jeweiligen Reizgebers St der Informationsübertragung und das letzte Bit zum Steuern des Abgebens eines Reizimpulses. Die Code-Zuordnung ist an einem Beispiel in Figur 4 angegeben. Die serielle bit-Folge wird in ein Schieberegister eingelesen, nachdem durch das Synchronisations-Bit ein Start-Impuls erzeugt wurde. Das Code-Wort wird mit einem fest verdrahteten Code verglichen. Stimmt das eingelesene Signal mit dem fest verdrahteten überein, so ist der jeweilige Reizgeber St angesprochen und der Ausgangs-Flip-Flop geschaltet, entweder "ein" oder "aus", je nachdem, ob das letzte Bit eine "1" oder "0" ist. Wenn der Ausgang von 0 auf 1 geht, wird ein Stimulationsimpuls von beispielsweise 500 Mikrosenkunden abgegeben und anschließend über die Stromquelle ein Speicherkondensator über eine Konstantstromquelle geladen, bis durch den Befehl "aus" der Ladevorgang nach beispielsweise 10 ms unterbrochen wird. Dabei zeigt sich während der Ladephase an der Elektrode 3 des entsprechenden Reizgebers (hier $St_1$) eine konstante Spannung 52, abhängig von der Größe des Gewebswiderstands. Grundsätzlich sind Vorrichtungen mit bis zu 100 und mehr Kanälen möglich. Der beschriebene und dargestellte zeitliche Ablauf stellt nur ein vorteilhaftes Beispiel dar.

Die Energieversorgung der Reizgeber $St_1$ bis $St_n$ und der Muskelpotentialsensoren $Ts_1$ bis $Ts_n$ über die Speiseeinrichtungen 6 und 12 erfolgt zweckmäßig in den Modulationspausen des Telemetriesenders 10. Die angesprochenen elektronischen Einzelschaltungen können grundsätzlich herkömmlich in bekannter Weise ausgelegt sein.

In einer praktischen Anwendung der Vorrichtung hat sich für einen Telemetriesender 10 eine Frequenz von 27, 12 MHz mit Puls-Code-Modulation bewährt, auf die auch die Empfänger 4 der Reizgeber St abgestimmt sind. Für die Sender 8 der Muskelpotentialsensoren Ts und den Telemetrieempfänger 9 wurde beispielsweise eine Frequenz von 40,68 bis 40,75 MHz bei einem Frequenzhub von jeweils 8 kHz gewählt, womit bis zu acht Reizgeber $St_1$ bis $St_8$ selektiv nach Maßgabe der datenverarbeitenden Schaltung 15 korrelierte Reizsignale abzugeben vermögen. Mit Vorteil können dabei zusätzliche und gleichartig ausgebildete Reizgeber $St_1$ bis $St'_1$ einem einzigen Übertragungskanal

eines Muskelpotentialsensors zugeordnet werden. In analoger Weise ist auch die Zuordnung mehrerer gleichartiger Muskelpotentialsensoren Ts zu einem Reizgeber St möglich.

Gegenüber Einrichtungen bekannter Art ist es mit der Einrichtung nach der Erfindung möglich, bei zentral-nervös bedingten Lähmungen und mit gewissen Einschränkungen auch bei peripheren Nerv-Läsionen, bei denen nicht alle Leitungsbahnen unterbrochen sind, so daß noch ein Rest von Willkürimpulsen zur Verfügung steht, diese zu verstärken und an therapeutisch ausgewählten Orten dem Muskel als supramaximalen Reiz für die Gesamtheit der ruhenden motorischen Einheiten zuzuführen.

Diese mit Hilfe der Rest-Aktionspotentiale ausgelöste Elektrostimulation erlaubt aufgrund der Verstärkung der Einzelkontraktionen des jeweiligen Muskels, beispielsweise Bewegungsvorgänge gegen die Schwerkraft oder statische Haltearbeit zu verrichten, was infolge der patnologisch geschwächten, reinen Willkürinnervation nicht mehr möglich wäre.

Darüberhinaus ist es mit Einrichtungen nach der Erfindung möglich, nur vorübergehend geschwächte Muskeln zum Zwecke der Wiedererlangung der ursprünglichen Funktion gezielt und dosiert über längere Zeit zu reizen, ohne den Patienten in seiner ohnehin eingeschränkten Bewegungsfreiheit zusätzlich einzuschränken, oder ihn mit erhöhten Infektionsrisiken zu belasten.

In diesem Sinne ist insbesondere auch die Möglichkeit zur gezielten "Erhaltungs-Stimulation" zu nennen, bei der vermittels einer Vorrichtung gemäß der Erfindung nicht mehr direkt innervierbare Muskelbereiche von intakten Muskeln des Patienten solange stimuliert werden, bis sie aufgrund eines Heilungsprozesses durch die ursprünglichen oder neue Leitungsbahnen versorgt werden können, oder bis die "Software" für ein kybernetisches Reizsystem für den betreffenden Patienten erstellt ist.

Deutsche Nemectron GmbH
Durlacher Allee 47

D-7500 Karlsruhe 1

13. Juli 1982
6455/82

PATENTANSPRÜCHE

1. Vorrichtung zur Stimulation eines menschlichen Muskels, mit mindestens einem im Muskel implantierbaren Reizgebern, mit einem Empfänger und einer Speiseeinrichtung, mit mehreren Elektroden zur Abgabe der Reizsignale, g e k e n n z e i c h n e t durch nachfolgende Merkmalskombination:

a) Mehrere separate in einem Muskel implantierbare Reizgeber ($St_1$ bis $St_n$) mit Elektroden (3), von denen jeder mit dem Empfänger (4) der ein für ihn bestimmtes Signal selektiv empfängt, mit einem Reizgenerator (5), dessen Reizsignal in seiner Amplitude, Frequenz und Dauer mittels eines Empfangssignals gesteuert ist, und mit einer Speiseein - richtung (6) versehen ist, die mit empfangener hochfrequenter Energie versorgt ist.

b) Implantierbare Muskelpotentialsensoren ($Ts_1$ bis $Ts_n$), von denen jeder mit einem Sender (8) versehen ist, der ein von

- 2 -

einem gemessenen Muskelpotential moduliertes und selektiv auswertbares Signal ausstrahlt und von denen jeder eine Speiseeinrichtung (12) aufweist, die ebenfalls mit empfangener hochfrequenter Energie versorgt ist.

c) Einen den Sendern (8) der Muskelpotentialsensoren ($Ts_1$ bis $Ts_n$) zugeordneten Telemetrieempfänger (9), der über eine datenverarbeitende Schaltung (15) einen den Reizgebern ($St_1$ bis $St_n$) zugeordneten Telemetriesender (10) moduliert.

2. Vorrichtung nach Anspruch 1, dadurch gekennzeichnet, daß die Speiseeinrichtungen (6 und 12) mit Sendeenergie des Telemetriesenders (10) betrieben sind.

3. Vorrichtung nach Anspruch 1 oder 2, dadurch gekennzeichnet, daß den Sendern (8) der Muskelpotentialsensoren ($Ts_1$ bis $Ts_n$) und dem Telemetrieempfänger (9) ein gegenüber den Empfängern (4) der Reizgeneratoren (5) und dem Telemetriesender (10) unterschiedliches Frequenzband zugeordnet ist.

4. Vorrichtung nach einem der vorhergehenden Ansprüche, dadurch gekennzeichnet, daß die Antennen (16 und 14) des Telemetrieempfängers (9) und des Telemetriesenders (10) in einer aufsetzbaren Einheit angeordnet sind, die die an einem den zu stimulierenden Muskel aufweisenden Körperglied unter mindestens partieller Bedeckung der Gliedmaße anbringbar ist.

5. Vorrichtung nach Anspruch 4, dadurch gekennzeichnet, daß die Antennen von Telemetrieempfänger und -sender in einer Manschette angeordnet sind, die das Körperglied umgeben kann.

6. Verfahren zur Stimulation eines menschlichen Muskels, wobei mindestens ein im Muskel implantierter Reizgeber mit Elektroden von einem Sender gesteuert wird, dadurch gekennzeichnet, daß mittels mehrerer an verschiedenen Orten im Muskel implantierter Muskelpotentialsensoren das jeweils gegebene Muskelpotential gemessen und über gleichfalls implantierter Sender an einen Telemetrieempfänger gesendet wird, das die empfangenen Signale in einer datenverarbeitenden Schaltung verarbeitet und die verarbeiteten Signale über einen Telemetriesender mehreren ebenfalls verteilt im Muskel implantierten, den Muskelpotentialsensoren zugeordneten Reizgebern übersandt wird, wodurch diese in ihrer Stärke und Dauer von den physiologischen Muskelaktionspotentialen gesteuerte Reizimpulse über Elektroden abgeben.

FIG.1

FIG.2

FIG.3

FIG. 4